Europäisches Patentamt

European Patent Office

Office européen des brevets

(19)

(11) **EP 0 929 347 B1**

(12) **EUROPÄISCHE PATENTSCHRIFT**

(45) Veröffentlichungstag und Bekanntmachung des Hinweises auf die Patenterteilung:
**27.10.2004 Patentblatt 2004/44**

(21) Anmeldenummer: **97941813.4**

(22) Anmeldetag: **22.08.1997**

(51) Int Cl.⁷: **A61N 7/00**

(86) Internationale Anmeldenummer:
**PCT/DE1997/001829**

(87) Internationale Veröffentlichungsnummer:
**WO 1998/007469 (26.02.1998 Gazette 1998/08)**

(54) **VORRICHTUNG ZUR BEHANDLUNG DES HERZENS**

DEVICE FOR TREATING THE HEART

DISPOSITIF POUR LA THERAPIE DU COEUR

(84) Benannte Vertragsstaaten:
**CH DE FR GB IT LI**

(30) Priorität: **22.08.1996 DE 19633985**

(43) Veröffentlichungstag der Anmeldung:
**21.07.1999 Patentblatt 1999/29**

(73) Patentinhaber: **STORZ MEDICAL AG**
**CH-8280 Kreuzlingen (CH)**

(72) Erfinder: **MARLINGHAUS, Ernst, H.**
**CH-8598 Bottighofen (CH)**

(74) Vertreter: **Witte, Alexander, Dr.-Ing. et al**
**Witte, Weller & Partner,**
**Patentanwälte,**
**Postfach 105462**
**70047 Stuttgart (DE)**

(56) Entgegenhaltungen:
**EP-A- 0 112 082    EP-A- 0 369 177**
**EP-A- 0 429 109    WO-A-93/19705**
**DE-A- 4 312 264    FR-A- 2 267 752**
**US-A- 3 951 140    US-A- 4 390 026**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

## Technisches Gebiet

Die Erfindung bezieht sich auf ein Therapieverfahren beispielsweise zur Behandlung des Herzens oder der Bauchspeicheldrüse, sowie auf eine Vorrichtung zur Durchführung dieses Verfahrens mit einer Ultraschallquelle, die fokussierte Ultraschallwellen erzeugt.

## Stand der Technik

Aus der DE 43 12 264 AI ist eine Therapievorrichtung zur Behandlung von Leiden des Herzens und herznaher Gefäße bekannt, bei der therapeutische Ultraschallwellen durch thermische Wirkung Gewebeveränderungen hervorrufen. Dabei wird insbesondere eine in den menschlichen Körper durch die Speiseröhre einführbare Ultraschallquelle verwendet.

Weiterhin ist es bekannt, zur Behandlung des Herzens Laser zu verwenden, deren Laserpulse insbesondere mit dem Herzrhythmus synchronisiert werden.

## Darstellung der Erfindung

Erfindungsgemäß ist erkannt worden, daß therapeutische Ultraschallwellen, die (lediglich) eine thermische Wirkung haben, nur sehr begrenzt zur Revaskularisation und zur Schmerztherapie dienen können. Darüberhinaus ist die Einführung eines Katheders mit der Ultraschallquelle durch die Speiseröhre für den Patienten unangenehm.

Andererseits ist der Aufwand, der für einen "Herz"-Laser erforderlich ist, sehr hoch. Weiterhin ist die Behandlung mit einem sogenannten Herz-Katheder, der beim Laser-Einsatz erforderlich ist, für den Patienten nicht ohne Risiko.

Der Erfindung liegt die Aufgabe zugrunde, eine Therapievorrichtung beispielsweise zur Behandlung des Herzens oder der Bauchspeicheldrüse anzugeben, die mit vergleichsweise geringem apparativen Aufwand und ohne Einführung eines Katheders eine effiziente Revaskularisation oder Schmerztherapie ermöglicht.

Eine erfindungsgemäße Lösung dieser Aufgabe ist im Patentanspruch 1 angegeben.

Das erfindungsgemäße Gerät eignet sich zur Behandlung des Herzens oder beispielsweise der Bauchspeicheldrüse, insbesondere zur Revaskularisation des Herzens.

Es verwendet eine Ultraschallquelle, die fokussierte Ultraschallwellen erzeugt. Die Ultraschallquelle ist Teil einer extrakorporai angeordnete Stoßwellenquelle.

Erfindungsgemäß ist nämlich erkannt worden, daß mit einer Stoß- bzw. Druckwellen erzeugenden Ultraschallquelle, wie sie beispielsweise bereits in der Lithotripsie eingesetzt wird, eine wesentlich effizientere Revaskularisation oder Schmerztherapie erzielt wird als mit einer Ultraschallquelle, die lediglich thermische Wirkungen erzeugt.

Insbesondere ist erfindungsgemäß erkannt worden, daß es - im Gegensatz zu der in der Literatur vertretenen Auffassung - durchaus möglich ist, eine Reihe von scheinbar durch Skelett-Bestandteile, wie Rippen "verdeckten" Organe effizient extrakorporal zu behandeln.

Beispielsweise für die Behandlung des Herzens gibt es ausreichend "Fenster" im Rippenbogen, um das Herz extrakorporal mit Ultraschallwellen behandeln zu können.

Die erfindungsgemäß eingesetzte Ultraschallquelle(n) kann so ausgebildet sein, wie dies beispielsweise in der EP-B-0 369 177 bzw. der inhaltsgleichen DE 38 35 318 C1 und der in beiden Druckschriften genannten Literatur beschrieben ist. Auf diese Druckschriften und die in dieser Druckschrift genannte Literatur wird im übrigen zur Erläuterung aller hier nicht näher beschriebenen Begriffe ausdrücklich verwiesen. Die Druckschrift EP-B-0 369 177 offenbart eine Vorrichtung gemäß der Präambel des Anspruchs 1. Der Anspruch 1 definiert die Vorrichtung der Erfindung.

Insbesondere kann die Stoßwellenquelle eine elektromagnetische Quelle mit wenigstens einer Spule aufweisen, wie dies in der vorstehend genannten DE 38 35 318 C1 beschrieben ist. Die Spule bzw. Spulen sind bevorzugt auf der Mantelfläche eines Zylinders oder eines Kegels angeordnet und beispielsweise als Zylinderspulen ausgebildet.

Bei dieser Spulenkonfiguration ist (wenigstens) ein Reflektor vorgesehen, der die annähernd senkrecht zur Achse der Spuleneinheit abgestrahlten Ultraschallwellen reflektiert. Dieser Reflektor kann - hierzu wird ebenfalls auf DE 38 35 318 C1 verwiesen - eine parabolische Kontur haben und die Stoßwellenquelle umgeben.

Soll ein vergrößerter Fokusbereich realisiert werden, so kann anstelle eines Reflektors mit paraboloider Kontur auch ein Reflektor verwendet werden, der in wenigstens einem Schnitt eine ellipsoide Form hat oder der als Konus mit "geraden" Schnitten ausgebildet ist.

Im Hinblick darauf, daß beispielsweise bei der Behandlung des Herzens die "Einschallung" der Ultraschallwellen durch "Fenster" im Rippenbogen erfolgen muß, ist der Reflektor nicht rotationssymmetrisch und die Fläche des Reflektors, durch die der Schall austritt, hat eine längliche Form. Die "längere" Achse der Schallaustritts-Fläche wird dann "parallel" zum Verlauf der Rippen angeordnet.

Zusätzlich kann das Koppelkissen, das zwischen Ultraschallquelle und Körper angeordnet ist, eine längliche Form haben, so daß die Einkoppelung der Schallwellen durch "Fenster" im Rippenbogen unterstützt wird.

Die Einschallung durch "Knochen-freie" Fenster im Rippenbogen wird dadurch unterstützt, daß wenigstens zwei extrakorporal angeordnete Ultraschallquellen vorgesehen sind, wobei (wenigstens) eine der (wenigstens) zwei Ultraschallquellen ein Linear-Ultraschallschwinger ist.

Bevorzugt ist ferner wenigstens eine Ortungseinrichtung vorgesehen, die insbesondere in eine Ultraschallquelle integriert ist.

Die Ortungseinrichtung kann eine Ultraschall-Ortungseinrichtung oder eine Röntgen-Ortungseinrichtung sein.

Weiterhin ist es bevorzugt, wenn ein Sensor vorgesehen ist, der die Herztätigkeit erfaßt, und dessen Ausgangssignal an eine Steuereinheit angelegt ist.

Um Bewegungs-Artefakte zu vermeiden, ist es von Vorteil, wenn die Steuereinheit den Aufbau des Bildes der Ortungseinrichtung mit der von dem Sensor erfaßten Herztätigkeit korreliert. Die Korrelation kann insbesondere mit der R-Zacke erfolgen.

Bei einer weiteren Ausgestaltung der Erfindung korreliert die Steuereinheit die Auslösung von Stoßwellen mit der Herztätigkeit.

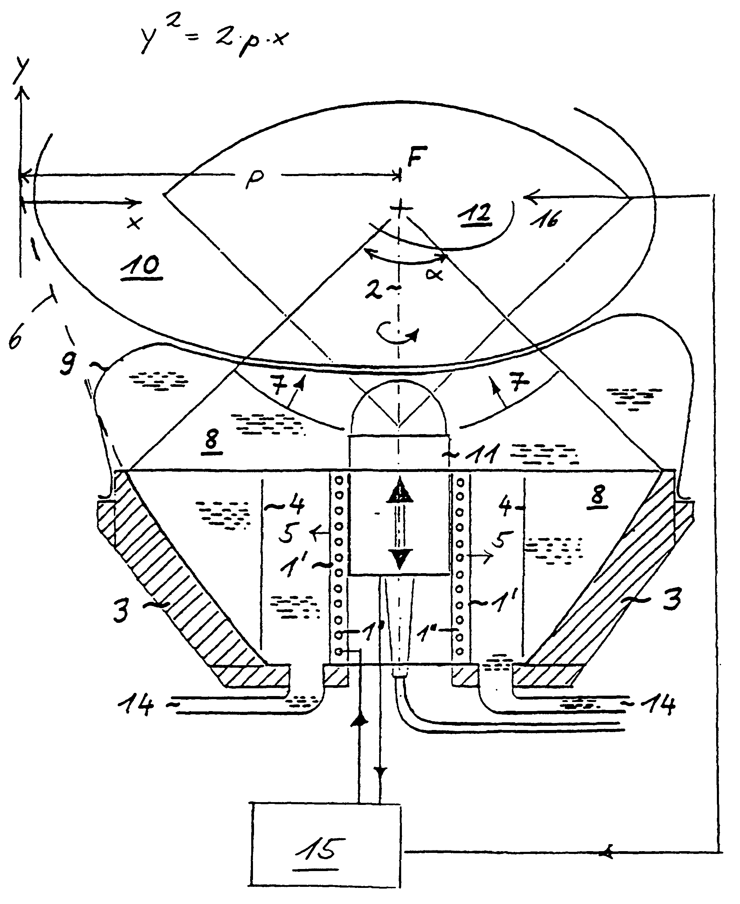

**Kurze Beschreibung der Zeichnung**

Die Erfindung wird nachstehend anhand eines Ausführungsbeispiels unter Bezugnahme auf die Zeichnung näher beschrieben, deren einzige Figur

einen Querschnitt durch eine erfindungsgemäße Stoßwellenquelle und eine Steuereinheit

zeigt.

In der Figur ist eine bevorzugt verwendete Stoßwellenquelle im Querschnitt sowie die zur Steuerung der Stoßwellenquelle verwendete Steuereinheit dargestellt.

Die Stoßwellenquelle weist eine Schallerzeugungseinheit 1 für das Therapiewellenfeld auf, deren Abstrahlfläche 1' bei dem gezeigten Ausführungsbeispiel die Form eines Zylinders hat. Koaxial zur Achse 2 der Stoßwellenquelle, die mit der Achse der zylinderförmigen Abstrahlfläche 1' zusammenfällt, ist ein Reflektor 3 angeordnet.

Der Reflektor 3 ist nicht rotationssymmetrisch, wobei seine Innenfläche in jedem Schnitt die Form einer Parabel hat. Ferner ist es möglich, Reflektoren zu verwenden, die in beiden Hauptschnitten unterschiedliche Formen haben:

So ist es möglich, daß beide Hauptschnitte zwar die Form von Parabeln haben, daß sich aber die Parabeln unterscheiden. Ferner ist es möglich, daß ein Hauptschnitt die Form einer Parabel, der andere aber die

Form einer Ellipse hat, so daß sich ein länglicher Fokusbereich ergibt, der beispielsweise bei der Therapie des Herzens aufgrund der Einstrahlungsbedingungen durch den von den Rippen freigelassenen Raum besonders vorteilhaft ist.

Da die Schallerzeugungseinheit 1 - wie nachfolgend noch erläutert werden wird - zylindrische Wellenfronten 4 erzeugt, die sich in Radialrichtung 5 von der Schallerzeugungseinheit 1 wegbewegen, ergeben sich nach Reflexion an dem Reflektor 3 mit insbesondere paraboloider Innenkontur fokussierte Wellenfelder 7 mit dem Aperturwinkel α, die im Fokuspunkt F (genauer gesagt "Fokusbereich") zusammenlaufen und dort Leistungsdichten erzeugen, die therapeutisch wirksam sind.

Die Parabel 6, die in dem dargestellten Schnitt des Reflektors 3 des gezeigten Ausführungsbeispiels vorliegt, ist durch die folgende Gleichung gegeben:

$$y^2 = 2px$$

wobei p der Abstand des Brennpunktes F vom Koordinatenursprung ist; die Lage des verwendeten Koordinatensystems ist der Figur zu entnehmen.

Ausdrücklich soll darauf hingewiesen werden, daß bei sehr hohen Amplituden der akustischen Therapiewellen deren Ausbreitungsverhalten vom linearen Ausbreitungsverhalten abweichen kann, so daß sich Veränderungen der Fokusgeometrie ergeben können; diese Veränderungen der Fokusgeometrie können durch entsprechende Anpassung der Reflektorfläche, beispielsweise durch geringfügige Abweichungen von der Parabelform kompensiert werden.

Ebenso können gezielte Änderungen des fokussierten Therapiewellenfeldes durch definierte Abweichungen von der Parabelform bis hin zur Ellipsenform und/oder durch eine entsprechende Änderung des Reflexionsverhaltens der Oberfläche erreicht werden.

Je nachdem, ob man eine Reflexion der Welle mit oder ohne Phasenumkehr anstrebt, wählt man ein Material für den Reflektor, das akustisch härter bzw. akustisch weicher ist als die Koppelflüssigkeit. Im Fall der Erzeugung von vorwiegend positiven Druckpulsen wird vorteilhafterweise eine Quelle, die positive Druckpulse erzeugt, und ein akustisch harter Reflektor verwendet. Je nach therapeutischer Zielsetzung können jedoch andere Quellen/Reflektorkombinationen sinnvoll sein.

Bei der Auswahl des Reflektormaterials ist ferner darauf zu achten, daß außer den bereits genannten Eigenschaften die Ausbreitungsgeschwindigkeit für transversale Oberflächenwellen kleiner oder nur geringfügig größer als die Ausbreitungsgeschwindigkeit der Therapiewellen in der Koppelflüssigkeit ist.

Bei dem dargestellten Ausführungsbeispiel ist ferner innerhalb der zylindrischen Abstrahlfläche 1' der Schallerzeugungseinheit eine Ultraschallortungseinrichtung 11, z.B. in Form eines Ultraschall-B-Bild-Geräts, angeordnet, die zur Darstellung der Fokuszone F im Körper eines Lebewesens 10 dient.

Weiterhin ist ohne Beschränkung des allgemeinen Erfindungsgedankens die Schallerzeugungseinheit exemplarisch als elektromagnetisches System dargestellt, das innerhalb der Abstrahlfläche 1 eine Spule 1" aufweist, die insbesondere eine Zylinderspule sein kann.

Es können selbstverständlich zur Schallerzeugung auch andere Elemente, beispielsweise piezoelektrische Quellen in Zylinderform verwendet werden.

Für die Einkoppelung akustischer Wellen in Körper von Lebewesen ist es vorteilhaft, die Wellen bereits in einem Medium mit gewebeähnlichen akustischen Eigenschaften zu erzeugen, um die Reflexionsverluste gering zu halten. Derartige Medien sind beispielsweise Wasser, Öle oder andere Flüssigkeiten, deren akustischen Impedanzen δ*c (Dichte * Schallgeschwindigkeit) der Impedanz von lebendem Gewebe im Einkoppelbereich der Therapiewelle nahekommt.

Weiterhin ist der Reflektor 3 von einer schalldurchlässigen, akustisch angepaßten Membran 9 abgeschlossen. In dem von dem Reflektor 3 und der Membran 9 gebildeten Raum 8 befindet sich eine Koppelflüssigkeit, so daß sich ein flexibles Kissen ergibt, das unter Vermeidung von Gaseinschlüssen akustisch an den Körper 10 angekoppelt wird.

Die Therapiewellen 4 breiten sich - wie bereits beschrieben - zunächst radial bezüglich der Rotationsachse 2 des Systems aus, und werden anschließend vom Reflektor 3 zum Fokuspunkt F reflektiert. Der akustische Reflektor besitzt dabei die vorstehend beschriebene Form und besteht aus einem Material mit einem hohen Reflexionsgrad in Bezug auf die Koppelflüssigkeit 8.

Das akustische fokussierte Wellenfeld 7 wird über die Membran 9 in den Körper 10 eingekoppelt und mit Hilfe der Ultraschall-Ortungseinheit 11 auf das therapeutische Zielgebiet 12, beispielsweise ein menschliches Herz ausgerichtet, das einer Revaskularisation unterzogen werden soll.

Die Ultraschallsonde 11 ist zur Optimierung der Bildqualität des Ultraschallbildes längs der Achse 2 in Pfeilrichtung verschiebbar angeordnet, so daß sie wahlweise bis an den Körper herangeschoben oder aber zur Vermeidung von Abschattungen des Therapiewellenfeldes zurückgezogen werden kann. Ein entsprechender Positionsmelder sorgt dafür, daß der Fokuspunkt des Therapiefeldes kontinuierlich im Ultraschallbild dargestellt wird. Weiterhin ist die Ultraschallsonde um die Achse 2 drehbar angeordnet, so daß das Ultraschallbild wahlweise verschiedene Schnittebenen zur Darstellung bringt.

Die Therapieeinheit besitzt neben den notwendigen Versorgungsanschlüssen für die Therapiequelle und die Ultraschallsonde Anschlüsse 14 für einen Volumenausgleich des Koppelkissens durch Zu- bzw. Ableitung geeignet präparierter Koppelflüssigkeiten, so daß eine akustisch günstige Anlegung der Membran 9 an den Körper 10 möglich wird. Die entsprechenden Versorgungsgeräte wie Pumpen, Steuereinheiten, Entgasungsvorrichtungen usw. sind nicht dargestellt.

Ferner ist einer Steuereinheit 15 vorgesehen, an die u.a. das Ausgangssignal der Ortungseinrichtung 11 angelegt ist. Ferner ist an die Steuereinheit 15 das Ausgangssignal eines Sensors 16 angelegt, der die Herztätigkeit erfaßt.

Die Steuereinheit 15 kann beispielsweise den Aufbau des Bildes der Ortungseinrichtung 11 mit der von dem Sensor 16 erfaßten Herztätigkeit und insbesondere mit der R-Zacke korrelieren. Ferner kann die Steuereinheit die Auslösung von Stoßwellen mit der Herztätigkeit korrelieren.

Vorstehend ist die Erfindung anhand eines Ausführungsbeispiels ohne Beschränkung des allgemeinen Erfindungsgedankens und der allgemeinen Anwendbarkeit der Erfindung beschrieben worden.

**Patentansprüche**

1. Vorrichtung zur Revaskularisation des Herzens oder zur Schnmerztherapie innerer Organe, wie der Bauchspeicheldrüse, mit wenigstens einer extrakorporal angeordneten Stoßwellenquelle, die Ultraschallwellen erzeugt und welche einen Reflektor aufweist, der die erzeugten Ultraschallwellen reflektiert and fokussiert,
   **dadurch gekennzeichnet**, daß der Reflektor nicht rotationssymmetrisch ist und daß die Fläche des Reflektors, durch die der Schall austritt, eine längliche Form hat.

2. Therapievorrichtung nach Anspruch 1,
   **dadurch gekennzeichnet, daß** die Stoßwellenquelle eine elektromagnetische Quelle mit wenigstens einer Spule ist.

3. Therapievorrichtung nach Anspruch 2,
   **dadurch gekennzeichnet, daß** die Spule bzw. Spulen auf der Mantelfläche eines Zylinders angeordnet sind, und daß ein Reflektor vorgesehen ist, der die abgestrahlten Ultraschallwellen reflektiert.

4. Therapievorrichtung nach Anspruch 3,
   **dadurch gekennzeichnet, daß** der Reflektor eine parabolische Kontur hat.

5. Therapievorrichtung nach Anspruch 3 oder 4,
   **dadurch gekennzeichnet, daß** der Reflektor die Stoßwellenquelle umgibt.

6. Therapievorrichtung nach einem der Ansprüche 1 bis 5,
   **dadurch gekennzeichnet, daß** die Therapievorrichtung ein Koppelkissen aufweist, das zwischen Ultraschallquelle und Körper angeordnet ist und eine längliche Form hat.

7. Therapievorrichtung nach einem der Ansprüche 1 bis 6,

**dadurch gekennzeichnet, daß** wenigstens zwei extrakorporal angeordnete Ultraschallquellen vorgesehen sind.

8.  Therapievorrichtung nach Anspruch 7, **dadurch gekennzeichnet, daß** eine der beiden Ultraschallquellen ein Linear-Ultraschallschwinger ist.

9.  Therapievorrichtung nach einem der Ansprüche 1 bis 8, **dadurch gekennzeichnet, daß** wenigstens eine Ortungseinrichtung vorgesehen ist.

10. Therapievorrichtung nach Anspruch 9, **dadurch gekennzeichnet, daß** die Ortungseinrichtung in eine Ultraschallquelle integriert ist.

11. Therapievorrichtung nach Anspruch 9 oder 10, **dadurch gekennzeichnet, daß** die Ortungseinrichtung eine Ultraschall-Ortungseinrichtung ist.

12. Therapievorrichtung nach Anspruch 9 oder 10, **dadurch gekennzeichnet, daß** die Ortungseinrichtung eine Röntgen-Ortungseinrichtung ist.

13. Therapievorrichtung nach einem der Ansprüche 1 bis 12, **dadurch gekennzeichnet, daß** ein Sensor vorgesehen ist, der die Herztätigkeit erfaßt, und dessen Ausgangssignal an eine Steuereinheit angelegt ist.

14. Therapievorrichtung nach Anspruch 15, **dadurch gekennzeichnet, daß** die Steuereinheit den Aufbau des Bildes der Ortungseinrichtung mit der von dem Sensor erfaßten Herztätigkeit korreliert.

15. Therapievorrichtung nach Anspruch 14, **dadurch gekennzeichnet, daß** die Steuereinheit den Bildaufbau mit der R-Zacke korreliert.

16. Therapievorrichtung nach einem der Ansprüche 12 bis **dadurch gekennzeichnet, daß** die Steuereinheit die Auslösung von Stoßwellen mit der Herztätigkeit korreliert.

**Claims**

1.  A device for the revascularization of the heart or for the pain therapy of inner organs, like the pancreas, comprising at least one extracorporeally arranged shock wave source, which generates ultrasonic waves, and which has a reflector reflecting and focusing the generated ultrasonic waves, **characterized in that** the reflector is not rotationally symmetrical and that the surface of the reflector through which the sound emerges has an elongate shape.

2.  The therapy device of claim 1, **characterized in that** the shock wave source is an electromagnetic source having at least one coil.

3.  The therapy device of claim 2, **characterized in that** the coil or coils is/are arranged on the peripheral surface of the cylinder, and that a reflector is provided which reflects the emitted ultrasonic waves.

4.  The therapy device of claim 3, **characterized in that** the reflector has a parabolic contour.

5.  The therapy device of claim 3 or 4, **characterized in that** the reflector surrounds the shock wave source.

6.  The therapy device of anyone of claims 1 through 5, **characterized in that** the therapy device comprises a coupling pad which is arranged between the ultrasonic source and the body and has an elongated shape.

7.  The therapy device of anyone of claims 1 through 6, **characterized in that** at least two extracorporeally arranged ultrasonic sources are provided.

8.  The therapy device of claim 7, **characterized in that** one of the two ultrasonic sources is a linear ultrasonic transmitter.

9.  The therapy device of anyone of claims 1 through 8, **characterized in that** at least one locating detector device is provided.

10. The therapy device of claim 9, **characterized in that** the locating detector device is integrated in an ultrasonic source.

11. The therapy device of claim 9 or 10, **characterized in that** the locating detector device is an ultrasonic locating detector device.

12. The therapy device of claim 9 or 10, **characterized in that** the locating detector device is an X-ray locating detector device.

13. The therapy device of anyone of claims 1 through 12, **characterized in that** a sensor is provided which detects the cardiac activity, and an output signal of which is applied to a control unit.

14. The therapy device of claim 13, **characterized in that** the control unit correlates the build-up of the image of the locating detector device with the cardiac activity detected by the sensor.

15. The therapy device of claim 14, **characterized in that** the control unit correlates the build-up of the image with the R-peak.

16. The therapy device of anyone of claims 12 through 15, **characterized in that** the control unit correlates the triggering of shock waves with the cardiac activity.

**Revendications**

1. Dispositif destiné à revasculariser le coeur ou pour traiter la douleur sur des organes internes, comme le pancréas, comprenant au moins une source d'ondes de choc extracorporelle, qui produit des ondes ultrasonores et présente un réflecteur, qui réfléchit et focalise les ondes ultrasonores produites, **caractérisé en ce que** le réflecteur n'est pas symétrique en rotation et **en ce que** la surface du réflecteur par laquelle sort le signal ultrasonore présente une forme allongée.

2. Dispositif thérapeutique selon la revendication 1, **caractérisé en ce que** la source d'ondes de choc est une source électromagnétique avec au moins une bobine.

3. Dispositif thérapeutique selon la revendication 2, **caractérisé en ce que** la bobine ou les bobines est/sont montée(s) sur la surface latérale d'un cylindre et **en ce qu'**est prévu un réflecteur qui réfléchit les ondes ultrasonores émises.

4. Dispositif thérapeutique selon la revendication 3, **caractérisé en ce que** le réflecteur présente un contour parabolique.

5. Dispositif thérapeutique selon la revendication 3 ou 4, **caractérisé en ce que** le réflecteur entoure la source d'ondes de choc.

6. Dispositif thérapeutique selon l'une des revendications 1 à 5, **caractérisé en ce que** le dispositif thérapeutique présente un coussin de couplage monté entre la source d'ondes ultrasonores et le corps, et qui présente une forme allongée.

7. Dispositif thérapeutique selon l'une des revendications 1 à 6, **caractérisé en ce que** sont prévues au moins deux sources d'ondes ultrasonores extracorporelles.

8. Dispositif thérapeutique selon la revendication 7, **caractérisé en ce que** l'une des deux sources d'ultrasons est un émetteur d'ultrasons linéaire.

9. Dispositif thérapeutique selon l'une des revendications 1 à 8, **caractérisé en ce qu'**est prévu au moins un dispositif de repérage.

10. Dispositif thérapeutique selon la revendication 9, **caractérisé en ce que** le dispositif de repérage est intégré dans une source d'ultrasons.

11. Dispositif thérapeutique selon la revendication 9 ou 10, **caractérisé en ce que** le dispositif de repérage est un dispositif de repérage à ultrasons.

12. Dispositif thérapeutique selon la revendication 9 ou 10, **caractérisé en ce que** le dispositif de repérage est un dispositif de repérage à rayons X.

13. Dispositif thérapeutique selon l'une des revendications 1 à 12, **caractérisé en ce qu'**il est prévu un capteur qui détecte l'activité cardiaque et dont le signal de sortie est appliqué à une unité de commande.

14. Dispositif thérapeutique selon la revendication 13, **caractérisé en ce que** l'unité de commande établit une corrélation entre l'élaboration de l'image du dispositif de repérage et l'activité cardiaque détectée par le capteur.

15. Dispositif thérapeutique selon la revendication 14, **caractérisé en ce que** l'unité de commande établit une corrélation entre l'élaboration de l'image et le pic R.

16. Dispositif thérapeutique selon l'une des revendications 12 à 15, **caractérisé en ce que** l'unité de commande établit une corrélation entre le déclenchement d'ondes de choc et l'activité cardiaque.

$$y^2 = 2 \cdot p \cdot x$$